# EUROPEAN PATENT APPLICATION

(11) **EP 0 635 494 A1**
(43) Date of publication of application: **25.01.1995**
(21) Application number: 94109475.7
(22) Date of filing: 20.06.1994
(51) Int. Cl.: C07D 233/61, A61K 31/415

(54) **2-(Imidazol-1-yl)2-benzylethylidene-aminooxyalkyl-hydroxamic acid derivatives**

(30) Priority: 20.07.1993 GB 9315058
(71) Applicant: PHARMACIA S.p.A., I-20152 Milano (IT)
(72) Inventor: Cozzi, Paolo, I-20133 Milan (IT); Giordani, Antonio, I-27100 Pavia (IT); Salvati, Patricia, I-20020 Arese (Milan) (IT); Bassini, Domenico Fusar, I-26010 Montodine (Cremona) (IT)
(74) Representative: Ferrario, Vittorino

(57) **Abstract**

A compound having the formula (I) 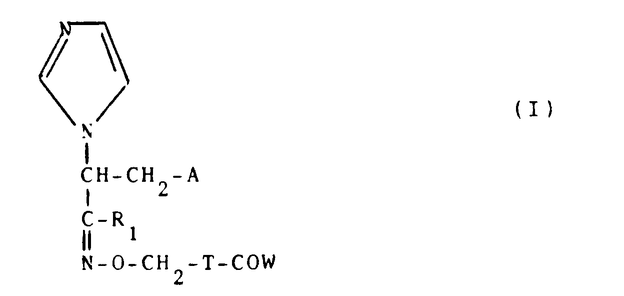
wherein
- A: is a phenyl ring unsubstituted or substituted by one to three substituents independently chosen from halogen, CF₃, C₁-C₄ alkyl and C₁-C₄ alkoxy;
- R₁: is phenyl unsubstituted or substituted by halogen, C₁-C₄ alkyl, C₁-C₄ alkoxy or CF₃; C₅-C₆ cycloalkyl; or a straight or branched C₁-C₆ alkyl group;
- T: is a branched or straight C₃-C₅ alkylene chain;
- W: is a group -OR or 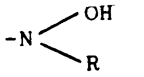 in which R is hydrogen or C₁-C₄ alkyl; or a pharmaceutically acceptable salt thereof; and wherein when, at the same time A is a phenyl ring unsubstituted or substituted either by C₁-C₄ alkoxy or by one or two substituents chosen from halogen and CF₃ and R₁ is a phenyl ring unsubstituted or substituted by halogen, CF₃ or C₁-C₄ alkoxy; C₅-C₆ cycloalkyl; or C₁-C₆ alkyl, then W is other than a group -OR as defined above,
which is useful as inhibitor of thromboxane A₂ (TxA₂) synthesis.

## Description

The present invention relates to new 2-(imidazol-1-yl)-2-benzylethylidene-aminooxyalkylhydroxamic acid derivatives, to the pharmaceutically acceptable salts thereof, to a process for their preparation, to pharmaceutical compositions comprising them and to their use as therapeutic agents. The present invention provides a compound having the general formula (I) 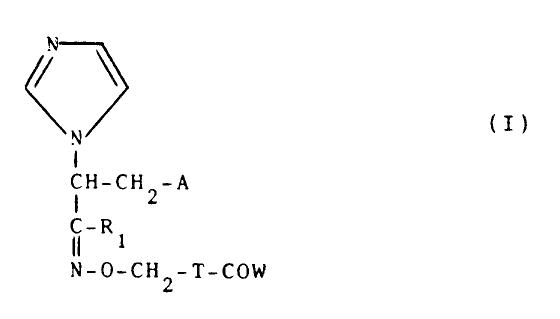
wherein
- A: is a phenyl ring unsubstituted or substituted by one to three substituents independently chosen from halogen, CF₃, C₁-C₄ alkyl and C₁-C₄ alkoxy;
- R₁: is phenyl unsubstituted or substituted by halogen, C₁-C₄ alkyl, C₁-C₄ alkoxy or CF₃; C₅-C₆ cycloalkyl; or a straight or branched C₁-C₆ alkyl group;
- T: is a branched or straight C₃-C₅ alkylene chain;
- W: is a group -OR or 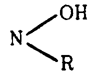 in which R is hydrogen or C₁-C₄ alkyl; or a pharmaceutically acceptable salt thereof, and wherein when, at the same time A is a phenyl ring unsubstituted or substituted either by C₁-C₄ alkoxy or by one or two substituents chosen from halogen and CF₃ and R₁ is a phenyl ring unsubstituted or substituted by halogen, CF₃ or C₁-C₄ alkoxy; C₅-C₆ cycloalkyl; or C₁-C₆ alkyl, then W is other than a group -OR as defined above.

The invention also includes within its scope all the possible isomers, stereoisomers and their mixtures and the metabolites and the metabolic precursors or bio-precursors of the compounds of formula (I).

In particular the compounds of formula (I) exhibit either E- or Z-isomerism about the oximic double bond. Both the single E- and Z-isomers of the compounds of formula (I) and their mixtures are also included within the scope of the present invention.

The alkyl and alkoxy groups may be branched or straight chain groups.

A C₁-C₆ alkyl group is preferably a C₁-C₄ alkyl group.

A C₁-C₄ alkyl group is e.g. methyl, ethyl, propyl, isopropyl, butyl or tert.butyl, more preferably methyl or butyl.

A C₁-C₄ alkoxy group is e.g. methoxy, ethoxy, propoxy, isopropoxy, butoxy or tert.butoxy, preferably methoxy, ethoxy or propoxy.

A halogen atom is suitably bromine, chlorine or fluorine, preferably it is bromine or fluorine.

T is preferably a divalent group chosen from -CH₂-CH₂-CH₂-

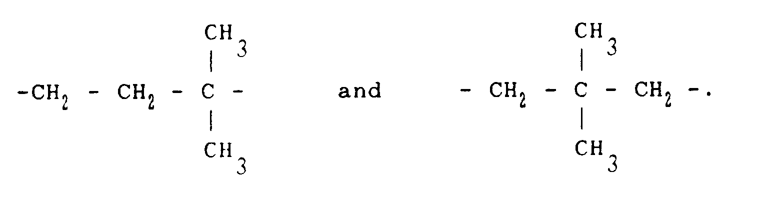

Pharmaceutically acceptable salts of the compounds of the invention include acid addition salts, with inorganic, e.g. nitric, hydrochloric, hydrobromic, sulphuric, perchloric or phosphoric acid, and organic, e.g. acetic, propionic, glycolic, lactic, oxalic, malonic, malic, maleic, tartaric, citric, benzoic, cinnamic, mandelic or salicylic acid, and salts with inorganic, e.g. alkali metal, especially sodium or potassium, bases or alkaline-earth metal, especially calcium or magnesium, bases, or with organic bases, e.g. alkylamines, preferably triethylamine.

As stated above, the present invention also includes within its scope pharmaceutically acceptable bio-precursors (otherwise known as pro-drugs) of the compounds of formula (I), i.e. compounds which have a different formula to formula (I) above but which nevertheless upon administration to a human being are converted directly or indirectly in vivo into a compound of formula (I).

The above proviso excludes from the scope of the present invention some related compounds which fall within the scope of the general definition given in WO 91/13062 and EP-A-0526200.

Preferred compounds of the invention are the compounds of formula (I) wherein, subject to the above proviso,
- A: is a phenyl ring unsubstituted or substituted by halogen or C₁-C₄ alkyl;
- R₁: is phenyl or cyclohexyl;
- T: is a defined above;
- W: is as defined above;
and the pharmaceutically acceptable salts thereof.

Examples of specific compounds according to the present invention are the following compounds, either as Z- or E-isomers or Z,E-mixtures of said isomers:
5-[2-benzyl-2-(imidazol-1-yl)-1-cyclohexylethylidene] aminooxypentanohydroxamic acid;
4-[2-benzyl-2-(imidazol-1-yl)-1-cyclohexylethylidene] aminooxybutanohydroxamic acid;
5-[2-benzyl-2-(imidazol-1-yl)-1-phenylethylidene]aminooxypentanohydroxamic acid;
5-[2-(4-fluorobenzyl)-2-(imidazol-1-yl-1-cyclohexylethylidene]aminooxypentanohydroxamic acid;
5-[2-(4-fluorobenzyl)-2-(imidazol-1-yl-1-phenylethylidene]aminooxypentanohydroxamic acid;
5-[2-(4-methylbenzyl)-2-(imidazol-1-yl)-1-cyclohexylethylidene]aminooxypentanoic acid; and the pharmaceutically acceptable salts thereof.

The compounds of the invention and the salts thereof can be obtained by a process comprising
a) reacting an oxime of formula (II) or a salt thereof 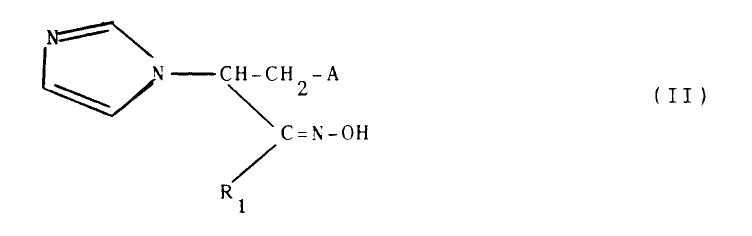 wherein
   A and R₁ are as defined above, with a compound of formula (III)

   Y-CH₂-T―COOR (III)

   wherein
   T and R are as defined above and Y is a leaving group; thus obtaining a compound of formula (I) in which W is a group -OR as defined above; or
b) reacting an oxime of formula (II) as defined above or a salt thereof with a lactone of formula (IV) 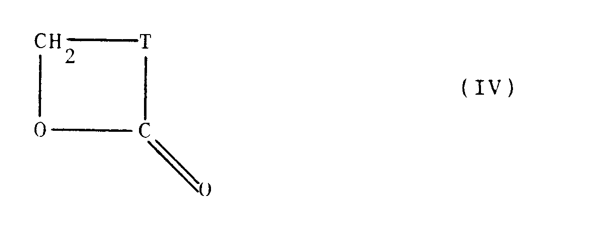 wherein
   T is as defined above, thus obtaining a compound of formula (I) in which W is -OH; or
c) reacting a compound of formula (V) 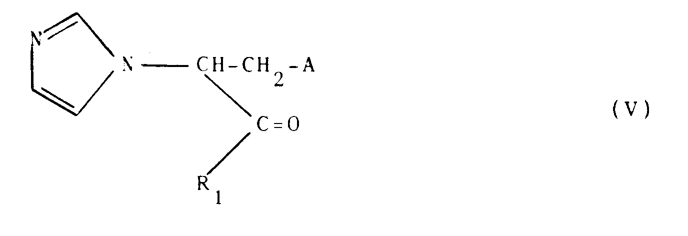 wherein
   A and R₁ are as defined above, with a compound of formula (VI)

   H₂N-O-CH₂-T―COOR (VI)

   wherein
   T and R are as defined above; thus obtaining a compound of formula (I) wherein W is an -OR group as defined above; or
d) reacting a compound of formula (V), as defined above, with a compound of formula (VII) 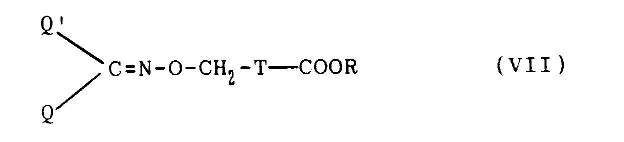 wherein
   T and R are as defined above and each of Q and Q' is independently hydrogen, lower alkyl or phenyl; thus obtaining a compound of formula (I) wherein W is an -OR group as defined above; or
e) reacting a compound of formula (VIII) 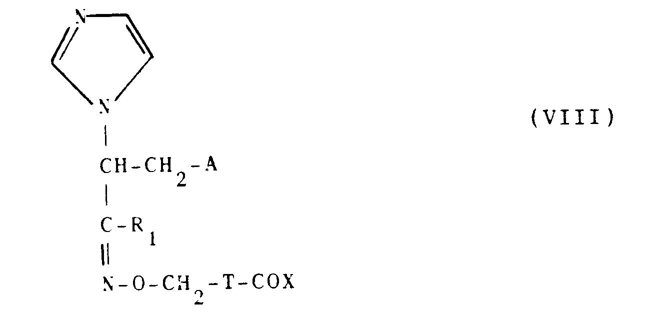 wherein
   A, R₁ and T are as defined above and X is a leaving group, with a compound of formula (IX) 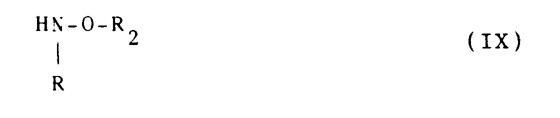 wherein
   R is as defined above and R₂ is hydrogen or a hydroxy-protecting group, and, if the case, removing the hydroxy-protecting group, thus obtaining a compound of formula (I) wherein W is a -N(OH)R group in which R is as defined above; and, if desired, converting a compound of formula (I) into another compound of formula (I), and/or, if desired, converting a compound of formula (I) into a salt thereof, and/or, if desired, converting a salt of a compound of formula (I) into a free compound, and/or, if desired, separating a mixture of isomers of a compound of formula (I) into the single isomers, and/or, if desired, altering by isomerization on the oxime double bond the ratio of E- and Z-isomers of a compound of formula (I) in a mixture thereof so as to obtain a different ratio of such isomers, and/or, if desired, converting by isomerization on the oxime double bond a pure E-isomer of a compound of formula (I) either into a pure Z-isomer thereof or into a mixture of E- and Z-isomers thereof; and/or, if desired, converting by isomerization on the oxime double bond a pure Z-isomer of a compound of formula (I) either into a pure E-isomer or into a mixture of E- and Z-isomers thereof.

A salt of a compound of formula (II) is for example an alkali metal salt, in particular a sodium or lithium salt.

A salt of a compound of formula (II) may be obtained according to known methods, for example a compound of formula (II) can be reacted with an alkali metal hydride, preferably NaH, in an inert organic solvent, e.g. dimethylformamide.

The leaving group Y in a compound of formula (III) is for example a halo group, in particular a chloro or bromo group, or a residue of an active ester group, in particular mesyl or tosyl.

The reaction of a compound of formula (II), or a salt thereof, with a compound of formula (III) can be carried out according to known methods, for example in the presence of an inert reaction organic solvent, e.g. dimethylformamide, dimethylsulfoxide, tert.butanol or benzene, and by addition of an appropriate basic agent e.g. an alkali metal carbonate, in particular sodium carbonate, or sodium hydride or potassium tert.butylate, at a temperature ranging from about 0°C to reflux temperature.

The reaction of a compound of formula (II) or a salt thereof, as defined above, with a compound of formula (IV) may be performed according to known methods. For example such reaction can be carried out by following the same reaction conditions described as to the reaction of a compound of formula (II), or a salt thereof, with a compound of formula (III).

The reaction of a carbonyl compound of formula (V) with an aminooxy derivative of formula (VI) can be carried out, for example, by dissolving the carbonyl compound in a reaction inert solvent, e.g. water, a lower alkanol, in particular ethanol, dioxane, tetrahydrofuran, an aromatic hydrocarbon, in particular benzene, toluene or xylene, or mixtures of such solvents, and by adding an appropriate basic agent, for example an alkali metal hydroxide, in particular sodium or potassium hydroxide, a carbonate or hydrogen carbonate, in particular the sodium and potassium ones, or an organic basic agent, e.g. a tertiary amine or pyridine.

When one or both of Q and Q' in a compound of formula (VII) is lower alkyl, it is for example C₁-C₄ alkyl, in particular methyl or ethyl.

Also the reaction of a compound of formula (V) with a compound of formula (VII) can be carried out according to known methods. For example such reaction can be performed in an inert reaction solvent, e.g. acetonitrile or acetic acid, and if required in the presence of a mineral acid, e.g. sulphuric or hydrochloric acid, at temperatures ranging from room temperature to reflux temperature.

In a compound of formula (VIII), the leaving group X may be any group capable of activating a carbonyl group in an acylation reaction. Accordingly, X may be for instance a halogen atom, preferably chlorine or bromine, imidazole, mesyloxy, tosyloxy or a lower alkanoyloxy group, typically acetoxy or trifluoroacetoxy.

A hydroxy protecting group in a compound of formula (IX) may be for instance on aryl-methyl group, typically a benzyl group.

The reaction of a compound of formula (VIII) with a compound of formula (IX) may be carried out according to known methods, for instance as described in Organic Chemistry, vol. 12, III, pages 406-432 (Academic Press).

In particular the reaction can be performed in an aprotic organic solvent, e.g., dichloromethane or tetrahydrofuran, in the presence of a suitable proton scavenger, e.g. an alkali metal salt, typically a potassium or sodium carbonate or hydrogen carbonate, or an organic basic agent, e.g. a tertiary amine or pyridine. The reaction can be performed at a temperature ranging from about -10°C to about 50°C, typically room temperature.

Removal of the hydroxy-protecting group R₂, thus providing a free hydroxamic acid of formula (I), can be carried out according to well known methods in the art. Typically when R₂ is an aryl-methyl group the de-blocking may be carried out by hydrogenolysis.

The conversion of a compound of formula (I) into another compound of formula (I) can be carried out by methods known in themselves.

For example, a compound of formula (I) containing an esterified carboxy group can be converted into the corresponding free carboxylic acid by known methods.

A compound of formula (I) containing a free carboxy group can be converted into a corresponding esterified carboxy derivative.

Such esterification reaction can be carried out according to known methods, preferably via an intermediate reactive derivative of the carboxylic acid, which may be isolated or not, by reaction with the appropriate C₁-C₄ alkanol.

The reaction can be carried out in a customary solvent e.g. benzene or toluene, or in the presence of an excess of the alkanol itself.

The reaction temperature may range from about 10°C to about 50°C. Intermediate reactive derivatives of the carboxylic acid may be for example acidic halides, e.g. the chloride, mixed anhydrides e.g. ethoxycarbonyl or tert.butyloxy anhydrides, or a suitable reactive intermediate obtained in situ e.g. by reaction with a diimide e.g., dicyclohexylcarbodiimide, or carbonyl diimidazole.

The optional salification of a compound of formula (I) as well as the conversion of a salt into the free compound and the separation of a mixture of isomers into the single isomers may be carried out by conventional methods.

For example the separation of a mixture of geometric isomers, e.g. Z- and E-isomers, may be carried out by fractional crystallization from a suitable solvent or by chromatography, either column chromatography or high pressure liquid chromatography.

The optional isomerization on the oxime double bond in a compound of formula (I), which is an equilibrium reaction, can be performed according to known methods; preferably in the presence of a mineral acid e.g. hydrochloric acid and/or by heating.

The oximes of formula (II) can be obtained according to known methods. For example a') by reaction of a compound of formula (V), as defined above, with hydroxylamine or an acid addition salt thereof, e.g. the sodium or potassium salt, or b') by reaction of an oxime of formula (X) 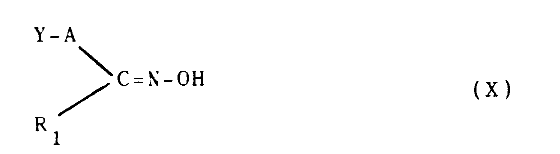
wherein Y, A and R₁ are as defined above, with imidazole, C₁-C₄ alkyl imidazole or a salt thereof, e.g. following for example the procedure in Arzneim. Forsch./Drug Res., 29(II), 1510-13, (1979).

In view of the oxime double bond, also an oxime of formula (II) may be obtained either as pure Z- or E-isomer or as a mixture thereof. Also an oxime of formula (II), if desired, can be submitted to the same isomerizations on the oxime double bond described above for a compound of formula (I), according to known methods. Similarly, a mixture of Z- and E-isomers of an oxime of formula (II) can be separated into the single isomers by following customary methods.

The compounds of formula (III), (IV) and (V) are either known compounds or can be obtained by known methods from known compounds. Also the compounds of formula (VI) are either known compounds or can be obtained from known compounds by following known methods, e.g. those described in Tetrahedron (1078), 23, 4441, or in general described in Organic Functional Group Preparation, by S.R. Sandler and W. Karo, Vol. III, chapter X, Academic Press (1972).

The compounds of formula (VII) can be obtained by reaction of a known compound of formula (XI) 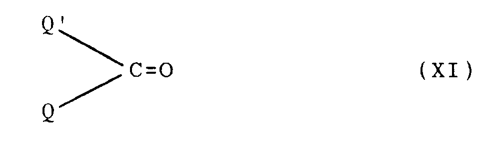
wherein Q and Q' are as defined above, with a compound of formula (VI) as defined above, by following the same reaction procedures described above under process c).

Alternatively a compound of formula (VII) can be obtained from a compound of formula (XI), via the corresponding oxime of formula (XII) 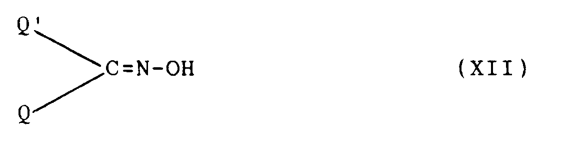
wherein Q and Q' are as defined above, by reaction with a compound either of formula (III) or of formula (IV) by following the same reaction conditions described above under processes a) and b).

The compounds of formula (VIII) can be obtained starting from the corresponding carboxylic acids of formula (XIII) or a salt thereof 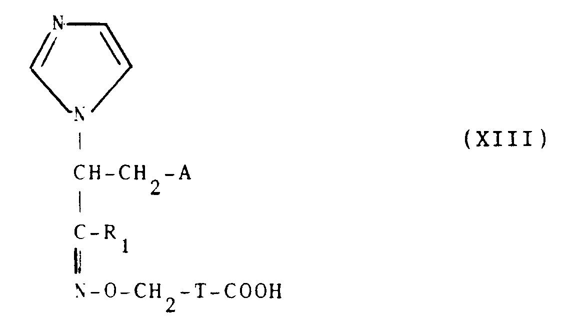
wherein A, R₁ and T are as defined above, according to known methods in the art. A salt of a compound of formula (XIII) may be an alkali metal salt, e.g. a lithium, sodium or potassium salt, or an ammonium salt, typically a triethylammonium salt.

A compound of formula (VIII) wherein X is chlorine can be for example obtained by reacting a compound of formula (XIII) or a salt thereof with thionyl chloride or oxalyl chloride.

The compounds of formula (IX), (X), (XI) and (XII) are known or can be obtained according to known methods.

The compounds of formula (XIII) are either new compounds according to the present invention wherein W is a group -OH, or they fall within the scope of WO 91/13062 (PCT/EP91/00351) and EP-A-526200 referred to above.

When in the compounds of the invention and in the intermediate products thereof groups are present which need to be protected during the reactions reported above, the groups can be protected in conventional way before the reaction takes place and then deprotected after its end, according to well known methods.

### PHARMACOLOGY

We have found that the compounds of formula (I), and the pharmaceutically acceptable salts thereof are selective inhibitors of thromboxane A₂ (TxA₂) synthesis and are therefore useful in the treatment of diseases related in particular to an enhancement of TxA₂ synthesis in mammals, including humans.

A human or animal patient may thus be treated by a method comprising the administration thereto of a therapeutically effective amount of a compound of formula (I) or a pharmaceutically acceptable salt thereof. The disease from which the patient is suffering can be ameliorated. The condition of the human or animal patient can thus be improved.

The compounds of formula (I) were for example tested for their ability to inhibit TxA₂ Synthase activity (as reflected by TxB₂ generated in whole blood during clotting or in isolated glomeruli) in vitro in the rat. The in vitro experiments were carried out as follows:
The effect of the compounds on TxA₂ synthesis was evaluated in serum.

Blood was withdrawn from the abdominal aorta of the animals under light ether anesthesia. The blood was immediately divided in portions of 0.5 ml and distributed in glass tubes each containing a concentration of the test compounds or of the reference compounds, i.e. Dazoxiben, which is a thromboxane synthase inhibitor (Randall et al. - Thromb. Res. 23, 145, 1981) and Acetylsalicylic Acid (ASA), which is a cyclooxygenase inhibitor.

Samples were then allowed to clot for 1 h at 37°C, centrifuged at 3000 rpm for 10 min, serum collected and stored at -20°C until assayed. TxB₂ levels were determined by RIA according to previously described procedures [Patrono et al. - Thromb. Res. 17, 3/4, 317, 1980] using highly specific antibody.

The compounds of the invention showed remarkable activity in the above tests.

In particular, for example, the compounds of the invention
(±)-(E)-5-[2-benzyl-2-(imidazol-1-yl)-1-cyclohexylethylidene]-aminooxybutanohydroxamic acid (internal code FCE 27790) and
(±)-(E)-5-[2-(4-methylbenzyl)-2-(imidazol-1-yl)-1-cyclohexylethylidene]aminooxypentanoic acid (internal code FCE 27818); and the pharmaceutically acceptable salts thereof were found to exhibit a marked inhibitory activity on TxA₂ synthesis significantly more potent than that of reference compound Dazoxiben.

These results are summarized in Table 1.

**Table I**

| In vitro effects on TxB₂ production in rat whole blood [Data are expressed as IC₅₀ (M) and limits for p=0.95]. | |
|---|---|
| Compound | Whole Blood (n = 8) |
| FCE 27790 | 7.10 x 10⁻⁷ (4.3x10⁻⁷- 13.9x10⁻⁷) |
| FCE 27818 | 1.38 x 10⁻⁷ (6.07x10⁻⁸ - 3.63x10⁻⁷) |
| Dazoxiben | 1.2 x 10⁻⁶ (0.70 - 1.9x10⁻⁶) |
| ASA | 4.2 x 10⁻⁵ (3.1 - 5.6x10⁻⁵) |

Wherein n is the number of replicants.

The compounds of the invention, being able to inhibit selectively the formation of TxA₂, can be used as vasodilatory and antiaggregant agents, for example in all the cases of thrombosis, peripheral vasculopathies and coronary artery disease. In fact, inhibition of TxA₂ production reduces the probability of thrombi formation and of vasoconstriction with consequent ischemic events and leaving unaltered (or increasing PGI₂ production, improves vasodilation, tissue blood supplies and protects the vessel wall.

Moreover, the compounds of the invention were tested for TxA₂ antagonism in a binding assay in washed human platelets, using as radiolabelled ligand [³H]-SQ 29,548. The experiments were carried out as follows:
Blood from healthy volunteers of both sexes who had not taken any medication for at least 10 days was collected into one-tenth volume of acid citrate dextrose containing indomethacin (28 µM). Platelet rich plasma (PRP), obtained by centrifugation of the blood at 200 x g for 20 min, was washed twice (1000 x g for 10 min). The platelets were then resuspended in Tyrode-Hepes buffer (pH 7.4) to a final concentration of 5-10x10⁻⁸ cells/ml and incubated for 0-60 min at 25°C with [³H]-SQ 29,548 (5 nM). For displacement experiments various concentrations (10⁻⁹ -10⁻⁴M) of competing ligands were added and incubated for 30 min at 25°C.

Non-specific binding was determined in the presence of 50 µM U46619 and was approximately 5% of total binding of [³H]-SQ 29,548. After incubation, 4 ml of ice-cold TRIS-HCl buffer (10 mM, pH 7.4) was added to each tube and the reaction mixture was immediately filtered by suction through a Whatman GE/C glass filter disc which was washed 2 times with ice-cold TRIS-HCl (4 ml) and counted for radioactivity by a Packard-β-counter.

The binding data were analyzed by computerized non-linear curve fitting using the Ligand program and expressed as IC₅₀. In Table II, as an example, the results obtained with the compounds of the invention coded as FCE 27790 and FCE 27818 in the binding test are compared to that obtained with the reference standard compound Sulotroban (BM 13177) [DE-A-2,809,377].

These results show that the representative compounds of the invention FCE 27790 and FCE 27818, besides being active as a TxA₂ synthase inhibitor, have also a good affinity for the receptor which is better than that shown by the known compound Sulotroban (BM 13177), which on the other hand is devoid of TxA₂ synthase inhibitory activity.

**Table II**

| ³H SQ 29,548 binding displacement (washed human platelets). IC₅₀ (M). | |
|---|---|
| BM 13177 | 7.3 x 10⁻⁶ |
| FCE 27790 | 3.6 x 10⁻⁷ |
| FCE 27818 | 6.94 x 10⁻⁸ |

Since the compounds of the present invention are both TxA₂ synthase inhibitors and PGA₂ (TXA₂) antagonists in the platelets on the basis of the state of the art, as reported e.g. in J. Clin. Invest. 80, 1435 (1987) and in Adm. Prostaglandins, Thromboxanes, Leukotrienes Res. Vol. 17 (1987) p. 49, these compounds are particularly suitable for the treatment of a disease state in which an enhancement of TxA₂ synthesis exerts a pathogenic effect, for instance in those mentioned above.

Another use of the compounds of the invention is for the treatment of migraine. It has been demonstrated that, in the case of migraine, a diffused vasoconstriction induced by platelet TxA₂ overproduction occurs.

[J. Clin. Pathol. (1971), 24, 250; J. Headache (1977), 17, 101].

A platelet overproduction of TxA₂ and MDA (malondialdehyde) in diabetes mellitus has been demonstrated and correlated with microcirculatory defects in the illness [Metabolism (1979), 28, 394; Eu. J. Clin. Invest. (1979), 9, 223; Thrombosis Haemat. (1979), 42, 983; J. Lab. Clin. Med. (1981), 97, 87]. Therefore, the compounds of the invention can be used in the treatment of diabetes, in particular, diabetic microangiopathy.

Moreover, the compounds of the invention can be used as anti-inflammatory agents. As is known, for example, fluid obtained from carrageenin-induced granuloma converts arachidonic acid into TxA₂ in vitro and TxA₂ levels are increased in the synovial fluid of rheumatoid arthritis patients and in the fluid of carrageenin-induced inflammation in rats [Prostaglands (1977), 13, 17; Scand. J. Rheum. (1977), 6, 151]. Recently it has been also demonstrated that an overproduction of TxA₂ is involved in the pathogenesis of hypertension and that a specific inhibitor of TxA₂ production may be employed in hypertension (Eu. J. Pharmacol. (1981), 70, 247). In fact, the compounds of the invention can be used as hypotensive agents. For example an increased TxA₂ synthesis and decreased prostacyclin synthesis are reported in pregnancy-induced hypertension [Am. J. Obstet. Gynecol. (1987), 157, 325; Hypertension (1988), 11, 550]. Treatment with thromboxane synthase inhibitors is therefore useful in this pathology.

Furthermore it has been shown that TxA₂ plays a role in the pathogenesis of ulcerative disorders of the stomach in accordance with its powerful gastric vasoconstrictory activity, so that also in this field a TxA₂ inhibitor is useful [Nature (1981), 202, 472]. In fact, the compounds of the invention are indicated for the treatment of peptic ulcers. The compounds of the invention can be also antitumoral agents. It is known, for example, that a selective inhibition of TxA₂ synthesis has been demonstrated to reduce the number of lung metastases and to slow down tumor growth [Nature (1982), 295, 188].

In view of the correlation between TxA₂ synthesis and calcium transport, recently showed by some authors, specific TxA₂ synthetase inhibitors, such as the compounds of the invention, can also find use in the treatment of osteoporosis, e.g. post-menopausal osteoporosis [Prostaglandins (1981), 21, 401].

Moreover, the compounds of the invention are indicated for the treatment of angina pectoris and heart failure.

In this respect, it is known, for example, that high levels of TxB₂ have been found in patients with Prinzmetal's angina [Prostaglandins and Med. (1979), 2, 243] and in patients with recurrent angina attacks [Sixth Intern. Congress on Thrombosis, Monte Carlo, October 1980, Abs. No. 140].

The platelet antiaggregatory activity of the compounds of the invention was evaluated in vitro and in vivo, for example, according to the modified methods of Born [Born G.V.R., Nature 194, 927 (1962)] and Silver [Silver M.J., Science 183, 1085 (1974)].

The compounds of this invention were found in vitro to have inhibitory activity on platelet aggregation induced by collagen or ADP (adenosine-5'-diphosphate) in platelet rich plasma of guinea pig [Dunkin Hantley Iva: PDH (SPF) Ivanovas GmbH, Germany].

Therefore the compounds of the invention may be useful in preventing or reducing platelet loss during extracorporeal circulation; for example during coronary artery bypass and graft procedures or during kidney dialysis. It has been, moreover, shown that circulatory shock, for example endotoxic and haemorrhagic shock, is associated with increased TxA₂ synthesis so that the compounds of the invention can be useful in these pathologies. Moreover, the compounds of the present invention can also be useful for the treatment of bronchial hyperreactivity in the therapy of asthma.

A role for TxA₂ in asthma can be inferred on the basis of its bronchoconstrictory activity in experimental animal models [Br. J. Pharmacol. (1984), 82 (3), 565]. An inhibitory activity of bronchospasm induced by Platelet Activating Factor (PAF) in rats is also reported, e.g. for the TxA₂ synthetase inhibitors described in GB-B-2205494.

The compounds of the present invention can also find use in the treatment of nephropathies e.g. forms of glomerulonephritis, diabetic nephropathy or nephropathies secondary to systemic lupus erythematosus (SLE), and in the prevention and/or treatment of Cyclosporin A-induced nephrosis.

Accordingly, the compounds of this invention can also be used for preventing and/or treating toxemia during pregnancy, typically preeclampsia, eclampsia and preeclamptic (eclamptic, esclamptogenic) toxemia.

Recently a positive correlation between enhanced intrarenal synthesis of TxA₂ and the progression of chronic glomerular disease has been demonstrated in different animal models of immune and non-immune renal damage and in humans [J. Clin. Invest. (1985), 75, 94; J. Clin. Invest. (1985), 76, 1011].

Accordingly, the TxA₂ synthase inhibitors recently described e.g. in GB-B-2205240 were found to be active in reducing proteinuria and creatinine serum levels in the doxorubicin induced nephrosis in rats and in reducing proteinuria and increasing the glomerular filtration rate (GFR) in the spontaneous focal glomerulosclerosis in the Milan Normotensive Strain (NMS) rats.

In particular in the treatment of renal failure the compounds of the invention may be used in association with an angiotensin converting enzyme inhibitor (ACEI), both as separate, sequential and substantially concomitant administration. The compounds of the invention can also be used to prevent or treat cyclosporin A-induced nephrosis in mammals.

The compounds of the invention may be also used to inhibit the renal and cardiac transplant rejection. In fact, after transplantation increased urinary TxB₂ excretion or whole blood TxA₂ synthesis have been reported both in man and rats [Lancet (1981), ii, 341; Transplantation (1987), 43, 346].

Another use of the compounds of the present invention is in the treatment of hyperlipidaemia, namely hypercholesterolaemia and hypertriglyceridaemia secondary to nephrotic syndrome.

Hyperlipidaemia is a common feature of nephrotic syndrome in man [New Engl. J. Med. (1983), 312 (24), 1544] and in addition elevated triglycerides and cholesterol levels are reported in animal models such as doxorubicin induced nephrotic syndrome [Expt. Mol. Pathology (1983), 39, 282]; elevated urinary albumin excretion has been suggested as the pathogenetic mechanisms [Kidney International (1987), 32, 813]. Also TxA₂ synthase inhibitors recently described in GB-B-2205240, e.g. proved to be active in reducing cholesterol and triglycerides in aged Milan Normotensive Strain rats and in reducing triglycerides in doxorubicin treated rats.

It has also been shown that in cholesterol fed rabbit, an animal model of diet induced atherosclerosis, arachidonic acid metabolism is an important factor in early lesion development. In particular a shift in metabolism from TxA₂ to PGE₂ may suppress lesion development (i.e. atheromatous plaque) in hypercholesterolaemia.

The compounds of the invention can be therefore used in this pathology.

The compounds of the invention can also be used in association with thrombolytic agents (e.g. tPA, Streptokinase, pro-Urokinase), in order to reduce the dose of the latter required in thrombolytic therapy, and to lower the incidence of reocclusion and possible haemorrhage.

A further application of the compounds of the invention is the prevention and/or treatment of restenosis after percutaneous transluminal angioplasty.

The toxicity of the compounds of the invention is negligible, therefore they can be safely used in therapy. Mice and rats which had been deprived of food for nine hours were treated orally with single administration of increasing doses of compounds of the invention, then housed and normally fed.

The orientative acute toxicity (LD₅₀) was assessed on the seventh day after the treatment.

In view of their high activity and low toxicity, the compounds of the invention can be safely used in medicine. The therapeutic regimen for the different clinical syndromes must be adapted to the type of pathology, taking into account, as usual, also the route of administration, the form in which the compound is administered and the age, weight and conditions of the subject involved.

The oral route is employed, in general, for all conditions requiring such compounds. Preference is given to intravenous injection or infusion for the treatment of acute pathological states.

For maintenance regimens the oral or parenteral, e.g. intramuscular, route is preferred.

The dosage levels suitable for oral administration to adult humans of the compounds of the invention e.g. FCE 27790 range from about 25 mg to about 300 mg per dose, 1 to 3 times a day.

Of course, these dosage regimens may be adjusted to provide the optimal therapeutic response.

The nature of the pharmaceutical compositions containing the compounds of this invention in association with pharmaceutically acceptable carriers or diluents will, of course, depend upon the desired route of administration.

The compositions may be formulated in the conventional manner with the usual ingredients. For example, the compounds of the invention may be administered in the form of aqueous or oily solutions, or suspensions, tablets, pills, gelatine capsules, syrups, drops or suppositories.

Thus, for oral administration, the pharmaceutical compositions containing the compounds of this invention are preferably tablets, pills or gelatine capsules which contain the active substance together with diluents, such as lactose, dextrose, sucrose, mannitol, sorbitol, cellulose; lubricants, for instance silica, talc, stearic acid, magnesium or calcium stearate, and/or polyethylene glycols; or they may also contain binders, such as starches, gelatine, methylcellulose, carboxymethylcellulose, gum-arabic, tragacanth, polyvinylpyrrolidone; disaggregating agents, such as starches, alginic acid, alginates, sodium starch glycolate; effervescing mixtures; dyestuffs; sweeteners; wetting agents, such as lecithin, polysorbates, laurylsulphates; and in general, non-toxic and pharmacologically inactive substances used in pharmaceutical formulations. Said pharmaceutical preparations may be manufactured in known manner, for example by means of mixing, granulating, tabletting, sugar-coating, or film-coating processes. The liquid dispersions for oral administration may be e.g. syrups, emulsions and suspensions. The syrups may contain as carrier, for example, saccharose or saccharose with glycerine and/or mannitol and/or sorbitol. The suspensions and the emulsions may contain as carrier, for example, a natural gum, agar, sodium alginate, pectin, methylcellulose, carboxymethylcellulose, or polyvinyl alcohol.

The suspensions or solutions for intramuscular injections may contain together with the active compound a pharmaceutically acceptable carrier, e.g. sterile water, olive oil, ethyl oleate, glycols, e.g. propylene glycol, and, if desired, a suitable amount of lidocaine hydrochloride. The solutions for intravenous injection or infusion may contain as carrier, for example, sterile water or preferably they may be in the form of sterile aqueous isotonic saline solutions.

The suppositories may contain together with the active compound a pharmaceutically acceptable carrier, e.g. cocoa-butter, polyethylene glycol, a polyoxyethylene sorbitan fatty acid ester surfactant or lecithin. The following examples illustrate but do not limit the present invention.

### Example 1

To a stirred solution of 1 g (0.0104 moles) of [1-cyclohexyl-2-(imidazol-1-yl)-2-(4-methylbenzyl)ethanone] in 50 ml of pyridine, 1.48 g (0.0095 moles) of 5-aminooxypentanoic acid hydrochloride are added at room temperature.

Stirring is continued for 8 hours.

The reaction mixture is evaporated under vacuum, diluted with water and acidified with acetic acid till pH=5.

The aqueous solution is extracted with methylene chloride, dried and evaporated. The residue is purified by column chromatography over silica gel (eluant: methylene-chloride/methanol 90/10).

The pure fractions are collected and evaporated, yielding 0.775 g of (±)(E)-5-[2-(4-methylbenzyl)-2-(imidazol-1-yl)-1-cyclohexylethylidene]aminooxypentanoic acid m.p. 140-2°C.

| Microanalysis: | | | |
|---|---|---|---|
| Found : | C 69.22 | H 8.04 | N 9.99 |
| Calculated for C₂₄H₃₃N₃O₃: | C 70.04 | H 8.03 | N 10.21 |

and 0.32 g of the corresponding Z-isomer:
(±)-(Z)-5-[2-(4-methylbenzyl)-2-(imidazol-1-yl)-1-cyclohexylethylidene]aminooxypentanoic acid:
m.p. 120-124°C

| Microanalysis: | | | |
|---|---|---|---|
| Found: | C 69.10 | H 8.25 | N 9.72 |
| Calculated for C₂₄H₃₃N₃O₃: | C 70.04 | H 8.03 | N 10.21 |

### Example 2

(±)-(E)-5-[2-benzyl-2-(imidazol-1-yl)-1-cyclohexylethylidene]aminooxypentanoic acid (400 mg, 1 mmol) was dissolved in dry chloroform (30 ml), and thionyl chloride (0.4 ml, 5.4 mmol) was added on stirring under dry nitrogen atmosphere. The resulting solution was stirred at 25°C for 2 hours, evaporated to dryness in vacuo and the residue, coevaporated twice front dry toluene was dissolved in THF (10 ml).

O-benzylhydroxylamine hydrochloride (1.6 g, 10 mmol) was suspended in water (50 ml) and solid NaHCO₃ (2 g) was added portionwise on stirring and cooling at 0°C. The reaction mixture was extracted twice with ethyl ether and the organic layers were dried (Na₂SO₄) and evaporated in vacuo. The resulting oil was dissolved in THF (10 ml) and added dropwise at 0°C to the solution of the acid chloride previously prepared.

The reaction mixture was stirred at 25°C for 2 hours, the solvent was evaporated and the residue dissolved in EtOAc (60 ml); the organic layer was washed with 10% NaHCO₃ solution, dried over Na₂SO₄ and evaporated to dryness in vacuo.

Column chromatography (eluant CH₂Cl₂-MeOH 98:2) afforded (±)(E)-5-[2-benzyl-2-(imidazol-1-yl)-1-cyclohexylethylidene]aminooxypentane O-benzyl hydroxamic acid (480 mg, 95%) as colorless oil.

This oil was dissolved in 95% ethanol (50 ml), 5% Palladium on charcoal (50 mg) was added and the resulting suspension was pressurized to 30 Psi with hydrogen for 50 min.

The catalyst was removed by filtration through celite and the filtrate evaporated in vacuo, the residue was crystallized from hexane-ethyl acetate to give (±)(E)-5-[2-benzyl-2-(imidazol-1-yl)-1-cyclohexylethylidene] aminooxypentanohydroxamic acid as colorless crystals (340 mg, 78%) melting at 90-91°C.

| Microanalysis: | | | |
|---|---|---|---|
| Calculated for C₂₃H₃₂N₃O₃: | C 66.96 | H 7.82 | N 13.58 |
| Found: | C 66.41 | H 7.91 | N 13.16 |

¹H-NMR: (400 MHz, CDCl₃)δ: 0.8-1.9 (m,14H, **CH₂CH₂**- + Cy-**H**), 2.0-2.3 (m,2H, CH₂COO), 2.97 (m,1H, Cy-H1), 3.18 (dd, J=9.1 Hz, J=13.9 Hz,1H, **H**_{**A**} Benzylic), 3.31 (dd, J=6.1 Hz, J=13.9 Hz,1H, **H**_{**B**} Benzylic), 4.0-4.3 (m,2H, OCH₂), 4.79 (dd,J=9.1, Hz, J=6.1 Hz, 1H,**CH**CH₂), 6.69-6.87 (two-s, 2H, H-4 H-5 imidazole), 6.9-7.3 (m,5H,Ar-H), 7.74 (s,1H, H-2 imidazole).

By proceeding analogously the following compounds can be obtained;
(±)(E)-4-[2-benzyl-2-(imidazol-1-yl)-1-cyclohexylethylidene]aminooxybutanohydroxamic acid.

| Microanalysis: | | | |
|---|---|---|---|
| Calculated for C₂₂H₃₀N₄O₃: | C 66.33 | H 7.54 | N 14.07 |
| Found: | C 65.06 | H 7.58 | N 12.43 |

¹H-NMR: (200 MHz, CDCl₃ δ: 0.7-2.3 (m,14H, Cy-H+ **CH₂CH₂**), 2.8-3.1 (m,1H, Cy-H1), 3.15 (dd, J=9.2 Hz, J=13.8 Hz, 1H, **H**_{**A**} Benzylic), 3.31 (dd, J=6.0 Hz, J=13.8 Hz,1H, **H**_{**B**} Benzylic), 4.0-4.3 (m,2H,OCH₂), 4.82 (dd, J=6.0 Hz, J=9.2 Hz,1H, **CH**CH₂), 6.8-7.3 (m,7H, Ar-H + H-5, H-6 imidazole), 7.58 (s,1H, H-2 imidazole).

5-[2-benzyl-2-(imidazol-1-yl)-1-phenylethylidene]aminooxypentanohydroxamic acid;
5-[2-(4-flurobenzyl)-2-(imidazol-1-yl)-1-cyclohexylethylidene]aminooxypentanohydroxamic acid; and
5-[2-(4-fluorobenzyl)-2-(imidazol-1-yl)-1-phenylethylidene]aminooxypentanohydroxamic acid.

### Example 3

To a solution of (±)(E)-5-[2-benzyl-2-(imidazol-1-yl)-1-cyclohexylethylidene]aminooxypentanohydroxamic acid (413 mg, 1 mmol) in 10 ml of methanol a methanolic solution of sodium methoxide (54 mg, 1 mmol) was added at 0°C. The reaction mixture was allowed to warm up to room temperature and evaporated in vacuo. The residue was dissolved in 15 ml of distilled water and lyophilized to give 43.5 mg of sodium salt, as a white powder.

### Example 4

Tablets, each weighing 150 mg and containing 50 mg of the active substance can be manufactured as follows:

| Composition (for 10,000 tablets) | |
|---|---|
| (±)-(E)-5-[2-benzyl-2-(imidazol-1-yl)-1-cyclohexylethylidene]aminooxypentanohydroxamic acid | 500 g |
| Lactose | 710 g |
| Corn starch | 237.5 g |
| Talc powder | 37.5 g |
| Magnesium stearate | 15 g |

(±)(E)-5-[2-benzyl-2-(imidazol-1-yl)-1-cyclohexylethylidene]aminooxypentanohydroxamic acid, lactose and a half of the corn starch are mixed: the mixture is then forced through a sieve of 0.5 mm openings. Corn starch (18 mg) is suspended in warm water (180 ml). The resulting paste is used to granulate the powder.

The granules are dried, comminuted on a sieve of sieve size 1.4 mm, the remaining quantity of starch, talc and magnesium are added, carefully mixed and processed into tablets using punches of 8 mm diameter.

## Claims

1. A compound having the formula (I) 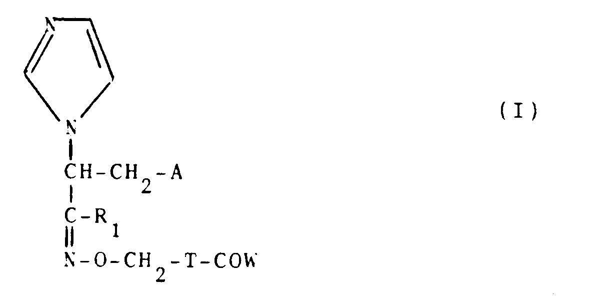 wherein
A is a phenyl ring unsubstituted or substituted by one to three substituents independently chosen from halogen, CF₃, C₁-C₄ alkyl and C₁-C₄ alkoxy;
R₁ is phenyl unsubstituted or substituted by halogen, C₁-C₄ alkyl, C₁-C₄ alkoxy or CF₃; C₅-C₆ cycloalkyl; or a straight or branched C₁-C₆ alkyl group;
T is a branched or straight C₃-C₅ alkylene chain;
W is a group -OR or 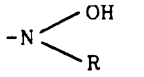 in which R is hydrogen or C₁-C₄ alkyl; or a pharmaceutically acceptable salt thereof; and wherein when, at the same time A is a phenyl ring unsubstituted or substituted either by C₁-C₄ alkoxy or by one or two substituents chosen from halogen and CF₃ and R₁ is a phenyl ring unsubstituted or substituted by halogen, CF₃ or C₁-C₄ alkoxy; C₅-C₆ cycloalkyl; or C₁-C₆ alkyl, then W is other than a group -OR as defined above.

2. A compound of formula (I), according to claim 1, wherein
A is a phenyl ring unsubstituted or substituted by halogen or C₁-C₄ alkyl;
R₁ is phenyl or cyclohexyl;
T is as defined in claim 1; and
W is as defined in claim 1;
or a pharmaceutically acceptable salt thereof.

3. A compound selected from the group consisting of
5-[2-benzyl-2-(imidazol-1-yl)-1-cyclohexylethylidene]aminooxypentanohydroxamic acid;
4-[2-benzyl-2-(imidazol-1-yl)-1-cyclohexylethylidene]aminooxypentanohydroxamic acid;
5-[2-benzyl-2-(imidazol-1-yl)-1-phenylethylidene] aminooxypentanohydroxamic acid;
5-[2-(4-fluorobenzyl)-2-(imidazol-1-yl-1-cyclohexylethylidene]aminooxypentanohydroxamic acid;
5-[2-(4-fluorobenzyl)-2-(imidazol-1-yl-1-phenylethylidene]aminooxypentanohydroxamic acid;
5-[2-(4-methylbenzyl)-2-(imidazol-1-yl)-1-cyclohexylethylidene]aminooxypentanoic acid; either as Z-or E-isomers or as mixtures of said isomers; and the pharmaceutically acceptable salts thereof.

4. A process for the preparation of a compound of formula (I), as defined in claim 1, or a pharmaceutically acceptable salt thereof, the process comprising
a) reacting an oxime of formula (II) or a salt thereof 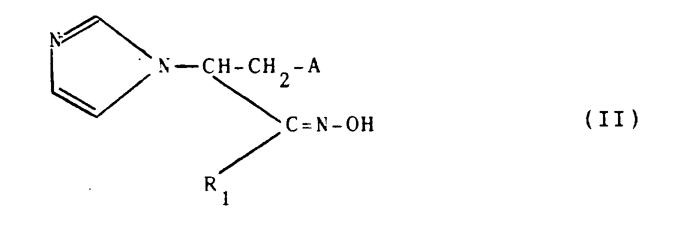 wherein
A and R₁ are as defined in claim 1, with a compound of formula (III)
Y-CH₂-T―COOR (III)
wherein
T and R are as defined in claim 1 and Y is a leaving group; thus obtaining a compound of formula (I) in which W is a group -OR as defined in claim 1; or
b) reacting an oxime of formula (II) as defined above or a salt thereof with a lactone of formula (IV) 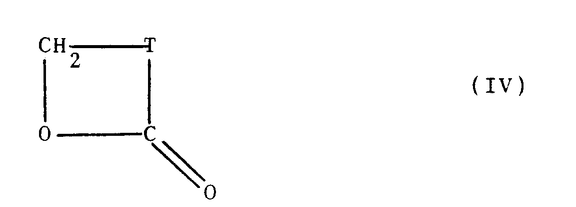 wherein
T is as defined in claim 1, thus obtaining a compound of formula (I) in which W is -OH; or
c) reacting a compound of formula (V) 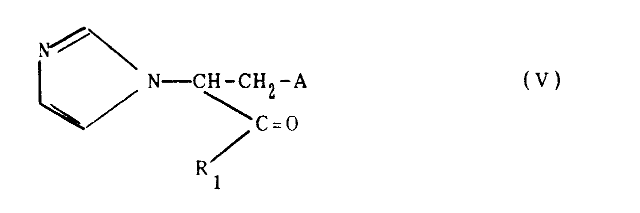 wherein
A and R₁ are as defined in claim 1, with a compound of formula (VI)
H₂N-O-CH₂-T―COOR (VI)
wherein
T and R are as defined in claim 1, thus obtaining a compound of formula (I) wherein W is an -OR group as defined in claim 1; or
d) reacting a compound of formula (V), as defined above, with a compound of formula (VII) 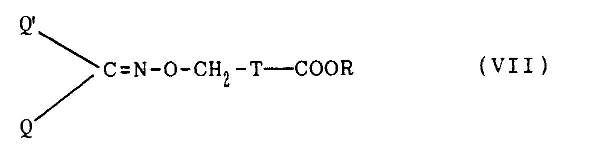 wherein
T and R are as defined in claim 1 and each of Q and Q' is independently hydrogen, lower alkyl or phenyl, thus obtaining a compound of formula (I) in which W is an -OR group as defined in claim 1; or
e) reacting a compound of formula (VIII) 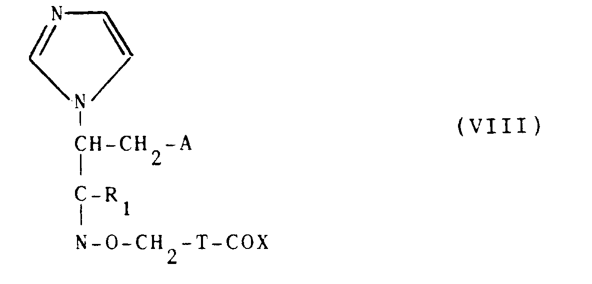 wherein
A, R₁ and T are as defined in claim 1 and X is a leaving group, with a compound of formula (IX) 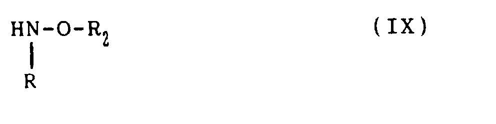 wherein
R is as defined in claim 1 and R₂ is hydrogen or a hydroxy-protecting group, and, if the case, removing the hydroxy-protecting group, thus obtaining a compound of formula (I) wherein W is a -N(OH)R group in which R is as defined in claim 1; and if desired converting a compound of formula (I) into another compound of formula (I), and/or, if desired, converting a compound of formula (I) into a salt thereof, and/or, if desired, converting a salt of a compound of formula (I) into a free compound, and/or, if desired, separating a mixture of isomers of a compound of formula (I) into the single isomers, and/or, if desired, altering by isomerization on the oxime double bond the ratio of E- and Z-isomers of a compound of formula (I) in a mixture thereof so as to obtain a different ratio of such isomers, and/or, if desired, converting by isomerization on the oxime double bond a pure E-isomer of a compound of formula (I) either into a pure Z-isomer thereof or into a mixture of E- and Z-isomers thereof; and/or, if desired, converting by isomerization on the oxime double bond a pure Z-isomer of a compound of formula (I) either into a pure E-isomer or into a mixture of E- and Z-isomers thereof.

5. A pharmaceutical composition comprising a suitable carrier and/or diluent and, as an active principle, a compound of formula (I) as defined in claim 1 or a pharmaceutically acceptable salt thereof.

6. A compound of formula (I) or a salt thereof, as defined in claim 1, for use in the treatment of a disease related to an enhancement of thromboxane A₂ synthesis.

7. A compound or salt as claimed in claim 6 for use in the treatment of nephropathies.

8. A compound or salt as claimed in claim 6 for use in the prevention and/or treatment of cyclosporin A-induced nephrosis.

9. A compound or salt as claimed in claim 6 for use in the treatment of hyperlipidaemia secondary to nephrotic syndrome.
